# EUROPEAN PATENT APPLICATION

(11) **EP 1 611 839 A1**
(43) Date of publication of application: **04.01.2006**
(21) Application number: 05253691.9
(22) Date of filing: 14.06.2005
(51) Int. Cl.: A61B 5/00

(54) **Methods of monitoring the concentration of an analyte**

(30) Priority: 30.06.2004 US 882642
(71) Applicant: Lifescan Scotland Ltd, Inverness IV2 3ED (GB)
(72) Inventor: Kraft, Ulrich, Hofheim 65719 (DE); Ebner, Manfred, Oberusel 61440 (DE); Steine, Matthias, Gilching 82205 (DE); McCluskey, Joseph, Sharon, Massachusetts 02067 (US)
(74) Representative: Tunstall, Christopher Stephen

(57) **Abstract**

Methods are disclosed for remotely monitoring an analyte concentration of an individual by one or more other individuals.

## Description

### BACKGROUND

There is a need to measure and monitor analyte concentrations in a continuous or in a frequent, periodic manner. For example, certain diabetics benefit from a system that can measure glucose concentration levels continuously and automatically without the need for human intervention. A variety of such systems exist, including those having sensors which are permanently or temporarily implantable or which establish continuous access to the patient's blood or interstitial fluid. Such systems provide diabetics with real-time glucose concentration levels.

It is contemplated that these systems include an alarm mechanism that is automatically activated to notify the user when his or her glucose level is outside of a physiologically normal zone. This would be especially useful for the nocturnal monitoring of diabetics. In such a scenario, when the patient enters a hypo or hyperglycemic state, the continuous glucose sensor activates an acoustical alarm (located either on the sensor itself or on a separate but closely positioned unit which is wired to or in wireless contact with the sensor) to wake up the diabetic person so that the appropriate therapy can be invoked. In certain cases, however, the alarm may not be sufficient to wake up the diabetic, particularly in situations where the diabetic is unable to be easily woken or has gone into a comatose state due to the hypo or hyperglycemic condition. Such an alarm is also not useful in situations where the diabetic is a baby or a very young child or is otherwise physically or mentally handicapped and unable to help himself in response to the alarm. In these situations, a parent or other caretaker must frequently and regularly check on the diabetic to monitor the diabetic's glucose level.

While wireless technologies are available to enable remote placement of an alarm, such as in the parent or caretaker's bedroom, due to Federal Communications Commission (FCC) regulations, these types of sensor systems are required to use a very low transmission frequency which limits placement of the alarm to no more than several meters from the sensor. Low frequency devices and specifically their antennas are necessarily relatively large. On the other hand, sensor-alarm systems capable of transmitting high frequency (above about 100 MHz) are subject to interference by the human body and, thus, have limited transmission range capacity, especially indoors. Additionally, high frequency wireless signals can consume large amounts of power requiring a battery size that limits portability of the alarm unit.

Accordingly, there is a continued need for the development of new devices and techniques for facilitating the remote monitoring of real-time analyte levels and other physiological characteristics that address the shortcomings of current technologies.

### SUMMARY

The present invention is directed to methods of monitoring analytes that satisfy the need to remotely monitor a patient and to remotely transmit patient data and/or to activate an alarm that obviates the drawbacks and shortcomings of prior systems. Further, the subject methods utilize systems which consume minimal power, provide relatively long-range signal transmissions and are less inclined to have interference with the human body than conventional analyte monitoring systems.

The analyte monitoring systems include a sensor for monitoring an analyte concentration of a user, a signal relay, and a signal receiver. In addition to monitoring analyte concentrations, the sensor is configured to transmit a first wireless signal to the signal relay which signal is representative of a real-time analyte concentration level, e.g., a value representative of a current glucose level, or a physiological state, e.g., hypo- or hyperglycemia. The signal relay is configured to receive the first wireless signal and to, in turn, transmit a second wireless signal to the signal receiver which is representative of such concentration level or state, wherein the second wireless signal has a different frequency and/or transmission protocol (i.e., including but not limited to signal transmission and reception times and data packaging (e.g., addressing, encoding, etc.)) than that of the first wireless signal. The signal receiver is configured to receive the second wireless signal and to provide notification to a user of the actual real-time analyte level, sensor state (function status, failure occurrence, error code, etc.) or a state representative thereof. Such notification may be an audible, tactile and/or visual alarm and may further include a display of the actual analyte concentration value. Accordingly, the analyte monitoring systems of the present invention can transmit an alarm by using a first frequency to communicate with the sensor to the relay over a relatively short distance, and subsequently using a second frequency to communicate with the relay to the receiving device over a relatively longer distance. The two signals may have the same or different frequencies. If the same frequency is used, the signals typically have different transmission protocols which do not interfere with each other.

A method of the present invention is directed to monitoring an analyte concentration of a first person by at least one secondary person. The method includes measuring the analyte concentration of the first person and transmitting a wireless signal representative of a real-time status of the analyte concentration value. A second wireless signal representative of the real-time status of the analyte concentration the concentration value having is then transmitted to the location of the at least one secondary person. The transmission range of the second signal is greater than the transmission range of the first signal.

Another method of the present invention is directed to relaying a real-time analyte concentration value of a first person by at least one secondary person. This method includes measuring the analyte concentration of the first person with a sensor and transmitting a first wireless signal representative of the real-time status of the analyte concentration from the sensor to a handheld device located a first distance from the sensor. A second wireless signal representative of the concentration value is then transmitted from the handheld device to a relay located a second distance from the sensor. A third wireless signal is then transmitted from the relay to at least one signal receiver located a third distance from the sensor, wherein the third wireless signal has a transmission range greater than the transmission range of the first wireless signal.

These and other objects, advantages, and features of the invention will become apparent to those persons skilled in the art upon reading the details of the invention as more fully described below.

### BRIEF DESCRIPTION OF THE DRAWINGS

The invention is best understood from the following detailed description when read in conjunction with the accompanying drawings:

Figure 1 is a schematic illustration of a first embodiment of an analyte monitoring system of the present invention.

Figure 2 is a schematic illustration of a second embodiment of an analyte monitoring system of the present invention.

### DETAILED DESCRIPTION OF THE DRAWINGS

Before the subject systems and method are described, it is to be understood that this invention is not limited to particular embodiments described or illustrated, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs.

It must be noted that as used herein and in the appended claims, the singular forms "a", "an", and "the" include plural referents unless the context clearly dictates otherwise. Thus, for example, reference to "a signal" includes a plurality of such signals and so forth.

All publications mentioned herein are incorporated herein by reference to disclose and describe the methods and/or materials in connection with which the publications are cited. The publications discussed herein are provided solely for their disclosure prior to the filing date of the present application. Nothing herein is to be construed as an admission that the present invention is not entitled to antedate such publication by virtue of prior invention. Further, the dates of publication provided might be different from the actual publication dates which may need to be independently confirmed.

Exemplary embodiments and variations of the present invention will now be described in detail. In further describing the present invention, the subject systems and device components will be described first. Next, various methods of using the subject devices and systems as well as methods for the transmission of real-time physiological information will then be described. Finally, a brief description is provided of the subject kits, which kits include the subject devices and systems for use in practicing the subject methods.

In the following description, the present invention will be described in the context of glucose concentration measurement; however, such is not intended to be limiting and those skilled in the art will appreciate that the subject devices, systems and methods are useful in the measurement and monitoring of other physical, neurological and chemical characteristics, *e.g*., blood pressure, heart rate, respiratory rate, neurological activity, therapeutic drug levels, fetal activity, sleep states, etc.

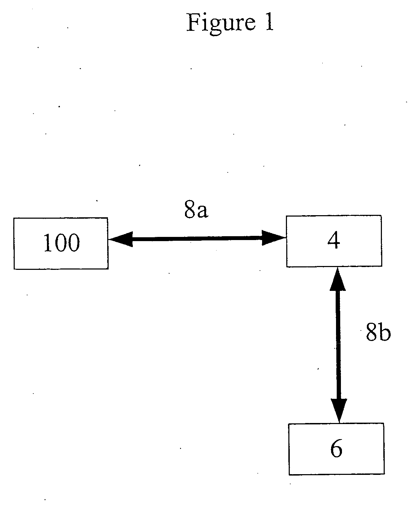

Figure 1 is a schematic representation of an embodiment of an analyte monitoring system of the present invention. Analyte monitoring system includes an analyte sensor 100, a signal relay 4, and a signal-receiving device 6.

Sensor 100 may be any suitable type of sensor, including but not limited to one that is permanently or temporarily implantable through or within subcutaneous, dermal, sub-dermal, intra-peritoneal or peritoneal tissue or is otherwise worn or attached to the body allowing continuous or intermittent measurement and access to the user's blood, interstitial fluid or the like. The sensors may be electrochemical, chemical or optical sensors or the like. Examples of such sensors which may be used with the present invention are disclosed in U.S. Patent Nos. 6,040,194; 6,232,130; 6,233,471; 6,272,364; 6,329,161; 6,514,718; 6,558,321 and 6,702,857, and in International Publication WO 02/49507, which are fully incorporated by reference herein. Examples of commercially available sensors usable with the present invention include but are not limited to Gluco Watch G2® Biographer from Cygnus, Inc., Redwood City, CA; CGMS® System Gold™ from Medtronic Minimed, Inc., Northridge, CA.

Sensor 100 may include an integrated signal transmitter or one that is directly coupled to the sensing portion of the sensor. The transmitter is preferably configured to transmit signals within the radio frequency (RF) spectrum. The sensor further includes a processor which may be programmed to enable the sensor to make continuous or intermittent but frequent measurements of the target analyte(s) and to transmit signals representative of those measurements continuously or intermittently. With non-implantable or partially implantable sensors, the sensor itself may also be configured to provide an alarm to the user to indicate a less than acceptable analyte measurement. With implantable sensors, the sensor may be configured to transmit a signal to activate an external alarm adjacent the user. Additionally, the sensor's processor may enable the detection of sensor malfunction, e.g., due to low battery power, temperature extremes, disconnection of the sensor from the user, etc., and the transmission of alarm signals representative of those malfunctions.

Signal relay 4 includes a signal-receiving portion configured to receive transmitted signals from sensor 100 and a transmitting portion configured to transmit signals to signal receiver 6. Again, the receiving and transmitting portions are preferably configured to operate within the RF band. Relay 4 is further configured to convert a received signal having one frequency and/or transmission protocol to a signal having another frequency and/or transmission protocol, and to transmit the converted signal having a transmission range greater than that of the received signal. Suitable relays which may be used with the present invention include those by Millenial Net, Inc. and ZigBee, Inc.

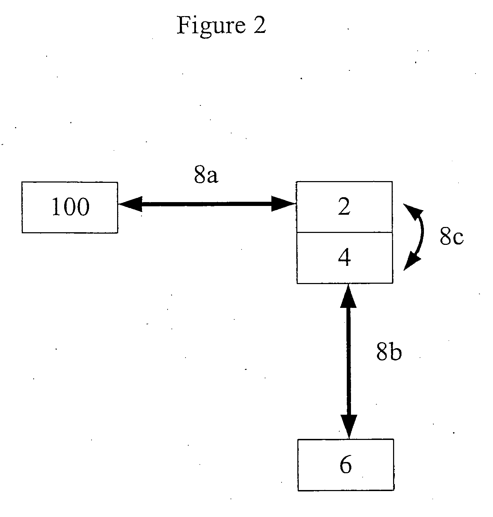

Depending on the user's setup, relay 4 may be used as a stationary and/or portable device. For example, relay 4 may be integrated into a substantially stationary base unit or station, as illustrated in Fig. 1, which may be powered by a designated power supply or by a wire or cable connection to a conventional AC outlet. With the relatively low energy signals transmitted by sensor 100, better results are achieved when the relay 4 is placed within about 3 meters from sensor 100. In one embodiment of this invention, relay 4 may be positioned in the room where a diabetic user resides. Alternatively, as illustrated in Fig. 2, relay 4 may be configured to interface with a handheld, battery-powered unit 2. Handheld device 2 may be configured to mate with relay 4 in a modular fashion using an electrical socket union such as a USB port wherein device 2 communicates information (signals) to relay 4 and relay 4 is powered by device 2. Alternately, relay 4 may be electrically integrated within handheld unit 2.

Handheld device 2 may also have the electronic functionality to measure an analyte concentration such as glucose in an episodic manner using a disposable glucose test strip. An example of an episodic glucose meter that can be incorporated into handheld device 2 is the commercially available LifeScan OneTouch® UltraSmart™ Monitoring System. Under certain situations it may be desirable for a system to measure glucose episodically in addition to the continuous method. For example, episodic glucose measurements may be needed to help calibrate sensor 100, perform a quality control check, make an emergency glucose measurement test while sensor 100 is equilibrating, or to confirm an extremely high or low measurement made by sensor 100 before taking drastic therapeutic actions. In another embodiment of the invention, handheld device 2 can be used as a remote control device sending and receiving data from sensor 100, an insulin pump (not shown), and other medical devices.

With any of the relay configurations described above, the base unit or handheld unit or both may include user interface controls for controlling sensor function as well as a display for displaying analyte values and other system parameters. The unit also typically includes a primary alarm, such as an audible, tactical (vibration) and/or visual (flashing LED) alarm signal, to notify the user of a critical or potentially critical state. Because relay 4 has an AC power source, it can generate a stronger alarm, e.g., a louder noise or a brighter light, than one that is generated solely from sensor 100 to help alert the diabetic user. Where sensor 100 is used in conjunction with an insulin pump as part of a closed-loop or feedback control system to control delivery of the appropriate dosage of insulin to maintain a euglycemic state, such an alarm may not be necessary. However, where such a closed-loop system is not employed, this primary alarm alone may not be sufficient to wake up a user when the user's glucose levels have reached a critical state.

Signal receiver 6 is configured to receive the higher energy signals from relay 4 and, as such, may be placed further away from relay 4 than the distance relay 4 is able to be placed from sensor 100, i.e., greater than about 3 meters. Receiving device 6 may be configured to be stationary whereby it is placed in a location or room (e.g., a bedroom, nurses' station) where a secondary person or user (e.g., parent, caregiver, nurse, etc.) is located. The stationary receiver may be battery powered or powered via an AC outlet source. Alternately, receiving device 6 may be a portable, battery-powered device which is configured to be worn or carried by the secondary person such as, for example, with a belt clip or on an armband.

With either of the signal receiver configurations, the receiver provides a secondary system alarm, such as an audible, a tactical (vibration) and/or a visual (e.g., one or more flashing light emitting diodes (LED)) alarm mechanism which is activated when the analyte concentration is outside of a physiological normal zone. In this way, the secondary user is immediately alerted to a critical or potentially critical state being experienced by the primary (e.g., diabetic) user. In one embodiment, an audible alarm may be configured to emit various volume (decibel) levels depending on the urgency or type of situation at hand. For example, a more urgent situation, e.g., the primary user's glucose levels have entered a physiological critical zone, would be provide a very loud alarm while. Alternatively, the alarm sound may be a recorded voice which literally announces the primary user's real-time status, e.g., "urgent", "caution", etc. Also, the type of sound may vary depending on the situation necessitating an alarm. For example, a beeping sound may be emitted for signaling the primary user's physiological status while a buzzing sound may be emitted for signaling a system problem, e.g., low battery, loss of signal reception, etc. Visual alarms may be configured to emit a plurality of colors, for example, where green indicates that the primary user is in a euglycemic state, yellow indicates that the primary user is in or entering a potentially hypo or hyperglycemic zone, and red indicates that the primary user's glucose level has entered unsafe hypo or hyperglycemic zone, where a blue light indicates a system failure or problem.

Receiving device 6 may further include a display, such as a liquid crystal display (LCD), which displays quantitative and/or qualitative real-time or stored (e.g., primary user information data about the primary user, e.g., a real-time measurement or several recently taken measurements of the primary user's glucose concentration. The display may also provide information regarding system parameters, e.g., remaining battery power, signal reception level, etc. As with the base unit or handheld unit associated with relay 4, signal receiver 6 may provide user interface controls such as functional menus, volume adjustment, etc.

So configured, the systems of the present invention enable wireless signals, i.e., alarm signals as well as information representative of analyte measurement and system operation parameters, to be transmitted to signal receiving device 6 from sensor 100 via signal relay 4. In other words, relay 4 is used as a conduit to transmit information to a person remotely located from a monitored individual. The system may include one or more additional signal receivers placed in different locations so as to transmit information to more than one person. In the context of the application discussed herein, the subject systems provide a convenient way to wirelessly alert one or more secondary persons about the glycemic status of a monitored diabetic.

Typically, the distance between the monitored individual and the secondary person is about 30 to 100 meters but may be more or less depending on the size of the building (e.g., home, hospital ward, etc) or area in which they users are located. Such a transmission range necessitates a signal transmission frequency that is greater than the allowable frequency range of sensor 100. Notwithstanding the federal regulations limiting medical sensor frequency ranges, practicality dictates that the size of sensor 100 be relatively small, e.g., no more than about a few cubic centimeters cubed, particularly if implanted, and thus having limited space capacity in which to house a battery or efficient antenna. Thus, only very small batteries having a low energy output are suitable for use with sensor 100. Due to the limited power supply, the range of signal transmission by sensor 100 is limited and the energy of the signals transmitted by sensor 100 is relatively low, e.g., no more than several hundred microwatts.

According to the present invention, signal relay 4 is employed to compensate for the limited range of transmission of sensor 100. As signal relay 4 is not implanted within the body, and in certain embodiments is not worn by the primary user, it does not have the size, space, transmission range and power constraints of sensor 100. As such, relay 4 is usable with a larger power supply source and is able to transmit signals at a higher energy over a longer distance. Although the higher energy is more susceptible to absorption by the body, the relay is remote enough to minimize such absorption.

Sensor 100 wirelessly communicates with relay 4 by means of a first transmission signal having a first frequency 8a and employing a first transmission protocol, and relay 4 wirelessly communicates with signal receiver 6 by means of a second transmission signal having a second frequency 8b and employing a second transmission protocol. If the same frequency is used for both, then the two transmission protocols are different, and visa-versa. Alternatively, both frequencies and both transmission protocols may be different. With any embodiment, first frequency 8a is sufficient to allow wireless communication from sensor 100 to relay 4 over a distance of no more than about 3 meters, and second frequency 8b is sufficient to allow wireless communication to occur between relay 4 and receiving device 6 over a distance greater than about 3 meters, and most typically up to about 30 to about 100 meters. Of course, the total transmission distance may be expanded as necessary by using one or more successively spaced relays.

In the embodiment of Fig. 2, handheld unit 2 may wirelessly communicate with relay 4 using a third transmission signal having a third frequency 8c employing a third transmission protocol sufficient to allow wireless communication to occur between unit 2 and relay 4 over a distance similar to the distance between sensor 100 and relay 4, but such distance may be greater or smaller. The third signal may have the same or a different frequency and/or utilize the same or a different transmission protocol as the first signal.

For practical reasons, signals within the radio frequency spectrum are preferable for applications of the present invention. Typically, the transmission signals used in the present invention have frequencies in the range from about 200 MHz to greater than 2.4 GHz. In one variation, the first and/or third frequencies 8a, 8c are typically in the range from about 200 MHz to about 950 MHz, and second frequency 8b is about 2.4 GHz (which enables the use of 802.11 wireless standards), but may be higher or lower as the application dictates. In one embodiment, either or both first and third frequencies are about 903 MHz (which frequency is available as part of the unlicensed spectrum of radio frequencies).

The wireless communication within the described systems may be entirely unidirectional, i.e., from the sensor to the relay to the receiver, or entirely bidirectional, i.e., the receiver may be able to transmit to the relay which is able to transmit to the sensor, or the systems may be partially unidirectional and partially bidirectional, e.g., communication between the sensor and relay or handheld unit may be bidirectional while communication between the relay and the signal receiver may be unidirectional. The frequencies of the signals transmitted in the opposite direction to what has been primarily described herein (i.e., transmissions from the receiver to the relay and from the relay to the sensor) may be the same or different from frequencies 8a, 8b and 8c, respectively.

The present invention further includes methods for monitoring an analyte concentration of a first person by at least one secondary person. In one variation, the method involves measuring the analyte concentration of the first person, such as with sensor 100 described above, and then transmitting a lower-energy wireless signal representative of a real-time status of the analyte concentration and/or an alarm reflective of such status to a relay station, such as with relay 4 described above (and/or to a handheld or base unit 2), where it is converted to a higher-energy wireless signal. The higher-energy signal is then transmitted to the secondary person at the location, and is received at the second location by of signal receiver, such as signal receiver 6 described above. An alarm on the signal receiver may be activated to alert the secondary person in response to an analyte concentration level of the first person which is outside an acceptable range. Additionally, the sensor and/or relay and/or handheld unit may have respective alarms which are activated under similar circumstances.

With the embodiment of Fig. 2, the first wireless signal may be sent simultaneously to both the handheld unit 2 and the relay 4, or may be sent to one or the other first which may then transmit a second wireless signal to the other. Typically, the first signal is sent to the handheld unit which in turn transmits it to the relay. As mentioned above, the handheld unit may transmit signals having the same or different energy as the sensor.

It is an advantage of this invention in that the first frequency range requires relatively low power and is less inclined to have interferences with the human body where sensor 100 is likely to be situated. A further advantage of the low power requirement is that it allows sensor 100 to have a smaller battery and/or less frequent battery charging/replacement which is highly desirable for both implanted and wearable continuous sensors. However, as discussed above, a lower energy signal generally causes the range of transmission to be limited. Relay 4 is thus used to relay the transmission of signals (i.e., information and data) from sensor 100 to receiving device 6.

It is a further advantage of the present invention in that second frequency 8b allows a much larger transmittal range. Although second frequency 8b requires relatively more power than first frequency 8a, the use of a stationary relay 4 using an AC power source or a large battery having higher energy output, thus, mitigating the power issue. It should be noted that the use of a higher-energy signal is more inclined to have physiological interferences with the human body, but this is typically not an issue as relay 4 is usually remote from a human body.

Also provided by the subject invention are kits for use in practicing the subject methods. The kits of one embodiment of the subject invention include at least one sensor, a relay and at least one receiver, as described above. The kits may further include software programs recorded on a CD-ROM or the like, which programs may be downloaded to the sensor, a base or handheld unit or meter, and/or a signal receiver by a user or a physician by means of an external device, such as a computer. Finally, the kits may further include instructions for using the subject devices. These instructions may be present on one or more of the packaging, label inserts or containers within the kits, or may be provided on a CD-ROM or the like.

It is evident from the above description and discussion that the above-described invention provides a simple and convenient way to wirelessly alert one or more secondary persons about the real-time glycemic status of a monitored diabetic. As such, the subject invention represents a significant contribution to the art.

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of the appended claims.

## Claims

1. A method of monitoring an analyte concentration of a first person by at least one secondary person, the method comprising:
measuring the analyte concentration of the first person;
transmitting a first wireless signal representative of a real-time status of the analyte concentration, the first signal having a first transmission range; and
transmitting a second wireless signal representative of the real-time status of the analyte concentration to the location of the at least one secondary person, the second wireless signal having a second transmission range;
wherein the second transmission range is greater than the first transmission range.

2. The method of claim 1, wherein a signal frequency of the first wireless signal is in the range from about 200 MHz to about 950 MHz and a signal frequency of the second wireless signal is about 2.4 GHz.

3. The method of claim 1 or 2, wherein the first wireless signal is transmitted about 3 meters or less and the second wireless signal is transmitted up to about 30 meters or more.

4. The method of claim 1, 2 or 3, further comprising activating an alarm at the location of the at least one secondary person in response to the real-time status of the analyte concentration.

5. The method of claim 4, further comprising activating an alarm at the location of the first person in response to the real-time status of the analyte concentration.

6. The method of any of the preceding claims, wherein in the analyte measuring is performed continuously.

7. The method of claim 6, wherein the analyte measuring is also performed episodically.

8. A method of relaying a real-time analyte concentration of a first person by at least one secondary person, the method comprising:
measuring the analyte concentration of the first person with a sensor;
transmitting a first wireless signal representative of a real-time status of the analyte concentration from the sensor to a handheld device located a first distance from the sensor, the first signal having a first transmission range;
transmitting a second wireless signal representative of the real-time status of the analyte concentration from the handheld device to a relay located a second distance from the sensor, the second wireless signal having a second transmission range; and
transmitting a third wireless signal representative of the real-time status of the analyte concentration from the relay to at least one signal receiver located a third distance from the sensor, wherein the third wireless signal has a transmission range than the transmission range of the first wireless signal.

9. The method of claim 8, further comprising activating an alarm at the location of the at least one secondary person in response to the real-time status of the analyte concentration.

10. The method of claim 8 or 9, wherein the third distance is substantially greater than the first distance.

11. The method of claim 8, 9 or 10, wherein the first distance and the second distance are substantially the same.

12. The method of claim 8, 9 or 10, wherein the third wireless signal has a signal frequency greater than a signal frequency of the second wireless signal

13. The method of claim 8, 9 or 10, wherein the third wireless signal has a transmission protocol different from a transmission protocol of the second wireless signal.
